# EUROPÄISCHE PATENTANMELDUNG

(11) **EP 1 378 261 A1**
(43) Veröffentlichungstag der Anmeldung: **07.01.2004**
(21) Anmeldenummer: 02021934.1
(22) Anmeldetag: 28.09.2002
(51) Int. Cl.: A61M 25/01

(54) **Vorrichtung zum Halten und Führen eines Führungsdrahtes in einem Katheter**

(30) Priorität: 05.07.2002 DE 20210509 U
(71) Anmelder: Scheu, Rolf Rainer, 60488 Frankfurt/Main (DE)
(72) Erfinder: Scheu, Rolf Rainer, 60488 Frankfurt/Main (DE)
(74) Vertreter: Müller-Gerbes, Margot, Dipl.-Ing.

(57) **Zusammenfassung**

Die Erfindung betrifft eine Vorrichtung zum Halten und Führen eines Führungsdrahtes in einem Katheter, welche an dem Führungsdraht lösbar befestigbar ist, mit einem proximalen und einem distalen Ende und mit einer vom proximalen Ende bis zum distalen Ende der Vorrichtung durchgehenden Ausnehmung für die Durchführung des Führungsdrahtes zum Einführen des Katheters in Gefäße oder Körperhöhlen eines Patienten, wobei sie Mittel zum lösbaren Befestigen des Katheters aufweist, so daß eine gemeinsame Bewegung des Führungsdrahtes und des Katheters bei vorher festgelegter Position des Führungsdrahtes in bezug auf den Katheter mittels der Vorrichtung ausführbar ist.

## Beschreibung

Die Erfindung bezieht sich auf eine Vorrichtung zum Halten und Führen eines Führungsdrahtes in einem Katheter, welche an dem Führungsdraht lösbar befestigbar ist, mit einem proximalen und einem distalen Ende und mit einer vom proximalen Ende bis zum distalen Ende der Vorrichtung durchgehenden Ausnehmung für die Durchführung des Führungsdrahtes zum Einführen des Katheters in Gefäße oder Körperhöhlen eines Patienten.

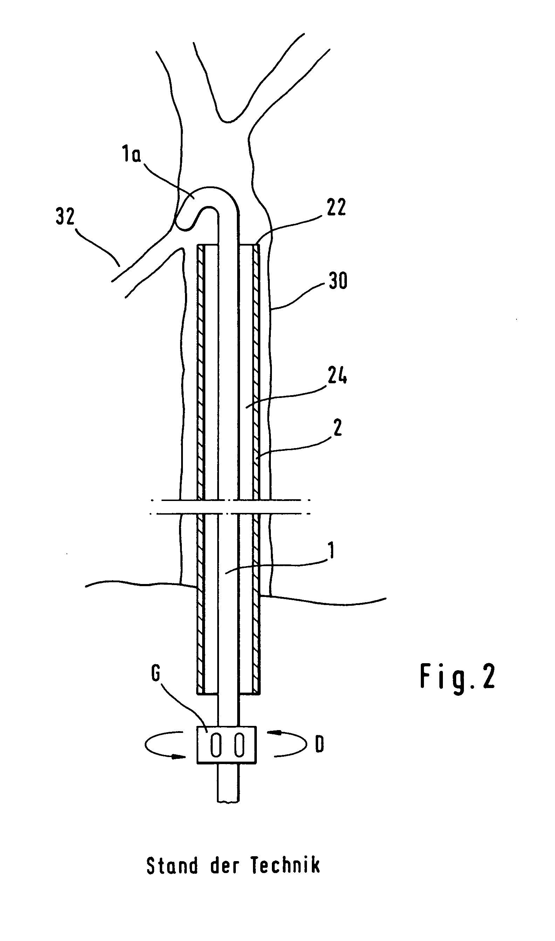

Führungsdrähte werden in Verbindung mit Kathetern vielfältig in der Medizintechnik angewendet, um Katheter z. B. in Gefäße eines Patienten einzuführen. Hierbei ist es bekannt, den Führungsdraht an seinem proximalen aus dem Katheter herausragenden Ende mit einer als Torque-Control bezeichneten Vorrichtung zum Fixieren und Halten zu versehen, wie aus der Figur 2 ersichtlich. Der mit der Halterung in Form des Drehgriffes G an seinem proximalen Ende versehene Führungsdraht 1, der durch das durchgehende Lumen 24 des Katheters 2 bis zu dessen distalem Ende 22 und darüber hinaus geführt ist, dient beispielsweise in der kardiologischen Angiographie zum Auffinden von Gefäßverzweigungen. Hierbei wird erst der Führungsdraht 1 in das Blutgefäß 30 vorgeschoben und dann der Katheter 2 über den Führungsdraht 1 in das Blutgefäß 30 vorgeschoben. Zum Auffinden und Einführen in eine Gefäßabzweigung 32 kann nun der Führungsdraht 1 durch Anfassen an den Drehgriff G in Pfeilrichtung D um seine Achse gedreht werden, wodurch sich dann auch die hier beispielsweise abgebogene Spitze 1a des Führungsdrahtes mitdreht und somit in die Abzweigung 32 eingeführt werden kann.

Nachteilig bei der als Torque-Control bekannten Vorrichtung zum Halten und Führen des Führungsdrahtes zwecks Plazierung eines Katheters oder einer Sonde ist, daß durch die relative axiale Verschieblichkeit von Führungsdraht zu Katheter eine vorgesehene Position des Führungsdrahtes sich während einer Behandlung verändern kann und ein sich im Katheter in axialer Richtung verschiebender Führungsdraht sowohl durch seitliche Augen am Katheter austreten kann oder auch Führungsdrähte, die keine abgebogene Spitze aufweisen oder zu weit am distalen Ende aus dem Katheter austreten, zu Verletzungsrisiken des Patienten führen.

Der Erfindung liegt die Aufgabe zugrunde, die Vorrichtung zum Führen und Halten eines Führungsdrahtes zum Einführen und Positionieren eines Katheters zu verbessern, dergestalt, daß unerwünschte axiale Verschiebungen von Führungsdraht und Katheter gegeneinander nicht möglich sind und damit auch Verletzungsrisiken verringert werden.

Diese Aufgabe wird erfindungsgemäß mit einer Vorrichtung gemäß den Merkmalen des Patentanspruches 1 gelöst.

Vorteilhafte Weiterbildungen der Erfindung sind den kennzeichnenden Merkmalen der Unteransprüche entnehmbar.

Unter Kathetern im Sinne der Erfindung werden auch Sonden verstanden.

Erfindungsgemäß wird eine Vorrichtung zum Halten und Führen eines Führungsdrahtes in einem Katheter vorgeschlagen, die Mittel zum lösbaren Befestigen des Katheters aufweist, so daß eine gemeinsame Bewegung des Führungsdrahtes und des Katheters bei vorher festgelegter Position des Führungsdrahtes in bezug auf den Katheter mittels der Vorrichtung ausführbar ist.

Erfindungsgemäß wird eine Katheter-Führungsdraht-Fixierung vorgeschlagen, die nicht nur zum Halten eines Führungsdrahtes dient, sondern gleichzeitig die Befestigung des Katheters an dieser Halterung ermöglicht. Eine solche Anordnung erlaubt ein gemeinsames gleichzeitiges Bewegen von Führungsdraht und Katheter, d. h. gemeinsame Positionsveränderung mit vorher festgelegter Position des Führungsdrahtes in bezug auf den Katheter und unter Beibehaltung dieser Position - auch beim gemeinsamen Bewegen -, so daß unerwünschte axiale Verschiebungen des Führungsdrahtes in bezug auf den Katheter nicht mehr auftreten können. Damit wird eine neue und wesentlich verbesserte und sichere Legetechnik für das Legen von Kathetern und Sonden mit Führungsdraht erreicht.

Die erfindungsgemäße Katheter-Führungsdraht-Fixierung dient also nicht nur zum Halten des Führungsdrahtes, sie hält gleichzeitig den Katheter bzw. die Sonde. Die gemeinsame Bewegung bewirkt einen Handhabungsvorteil für die Vorrichtung. Gleichzeitig verändert sich die einmal fixierte Position des Führungsdrahtes und damit der Führungsdrahtspitze in bezug auf den zugehörigen Katheter nicht. Dies dient insbesondere zur Vermeidung von Verletzungsrisiken durch den Führungsdraht. Bei der bekannten Vorrichtung gemäß Figur 2 kann ein sich im Katheter verschiebender Führungsdraht gegebenenfalls durch seitliche Augen eines Katheter oder wenn er eine offene Spitze hat auch durch diese unerwünscht austreten.

Erfindungsgemäß wird eine Fixierungsvorrichtung zum Durchführen und Fixieren des Führungsdrahtes vorgesehen, umfassend ein Gehäuse, ein in das Gehäuse einsetzbares Quetschteil und ein mit dem Gehäuse lösbar verbindbares Schließteil und die Mittel zum lösbaren Befestigen des Katheters am distalen Ende der Fixierungsvorrichtung angeordnet sind.

Als Mittel zum lösbaren Befestigen des Katheters wird vorgeschlagen, am distalen Ende der Fixierungsvorrichtung einen Katheteradapter mit Katheterkonnektor für die Konnexion des Katheters lösbar zu befestigen.

Nach einem weiteren Vorschlag der Erfindung ist es aber auch möglich, einen Katheterkonnektor für die Konnexion des Katheters integral mit der Fixierungsvorrichtung auszubilden, und zwar an dem distalen Ende der Fixierungsvorrichtung direkt als Katheterkonnektor auszubilden.

Der Katheteradapter mit Katheterkonnektor für die lösbare Befestigung an der Fixierungsvorrichtung bzw. die Anformung oder Ausbildung eines Katheterkonnektors unmittelbar an der Fixierungsvorrichtung kann in vielfältiger Form und bekannter Form ausgebildet sein, es können alle üblichen Katheterkonnektoren vorgesehen sein, wie beispielsweise die Ausbildung des Katheterkonnektors als Schlauchtülle oder als männlicher oder weiblicher Luer-Lock-Konnektor oder als Stufenkonnektor.

Die Fixierungsvorrichtung für den Führungsdraht wird erfindungsgemäß ein als Griff ausgebildetes Gehäuse mit einer durchgehenden Bohrung zum Durchführen des Führungsdrahtes, ein Quetschteil mit einer durchgehenden Bohrung zum Durchführen des Führungsdrahtes und ein als Stempel zum Einwirken auf das Quetschteil ausgebildetes Schließteil mit einer durchgehenden Bohrung vorgeschlagen, wobei das Quetschteil in die Bohrung des Gehäuses einsetzbar ist und das Schließteil an dem Gehäuse unter Ausübung eines Druckes auf das Quetschteil lösbar befestigbar ist, wobei die Bohrungen des Gehäuses und des Quetschteils und des Schließteils sich in einer gemeinsamen Achse erstrecken.

Das distale Ende der Fixierungsvorrichtung wird von dem Schließteil gebildet. Gemäß dem einen Vorschlag der Erfindung wird für die Befestigung und Halterung des Katheters an der Fixierungsvorrichtung ein Katheteradapter mit Katheterkonnektor vorgesehen, der an dem distalen Ende der Fixierungsvorrichtung, nämlich dem distalen Ende des Schließteils befestigbar ist. Hierfür wird vorgeschlagen, das distale Ende des Schließteils als Anschluß für die Konnexion eines Katheter-adapters auszubilden, beispielsweise als Luer-Lock-Anschluß.

Der Katheterkonnektor ist an dem Katheteradapter angeformt für die lösbare Variante.

Gemäß einem weiteren Vorschlag der Erfindung wird kein separater Katheteradapter für die Halterung des Katheters vorgesehen, sondern das distale Ende der Fixierungsvorrichtung, hier das distale Endes des Schließteils selbst, wird als Katheterkonnektor ausgebildet. Der Katheterkonnektor ist unmittelbar am distalen Ende des Schließteils angeformt und bildet dann eine Einheit mit dem Schließteil. Der Anschluß des Katheters an das integral mit einem Katheterkonnektor ausgebildete Schließteil kann dann wiederum beispielsweise über eine am Schließteil ausgebildete Schlauchtülle oder einen Luer-Lock-Konnektor oder Ausbildung als Stufenkonnektor erfolgen.

Das Schließteil, an dessen distalem Ende der Katheteradapter lösbar befestigt ist oder dessen distales Ende selbst als Katheterkonnektor ausgebildet ist, wird an seinem proximalen Ende mit dem Gehäuse lösbar verbunden, beispielsweise mittels einer Schraubverbindung, mittels eines Bajonettverschlusses und/oder mittels einer Rast- und/oder Klemmverbindung.

Das Gehäuse für die Fixierungsvorrichtung ist bevorzugt zylindrisch oder ballig ausgebildet und außenseitig zur Verbesserung der Griffigkeit mit einer Profilierung wie axial verlaufenden vorstehenden gleichmäßig über den Umfang verteilt angeordneten Rippen oder Riffelung ausgebildet. Zum Verbinden des Gehäuses mit dem Schließteil ist dieses im distalen Endbereich beispielsweise mit einem gegenüber dem Gehäuse abgesetzten und verkleinerten Durchmesser versehenen Bund ausgebildet, wobei der Bund außenseitig eine Profilierung wie Gewinde, Nuten, Wülste und/oder Rillen zur Ausbildung eines Formschlusses mit dem Schließteil aufweist.

Gemäß der Erfindung wird vorgeschlagen, das Schließteil mit einem von seinem proximalen Ende bis zu seinem distalen Ende durchgehenden Rohrstutzen mit durchgehender Bohrung auszubilden, wobei der proximale Endbereich des Rohrstutzens zum Einführen in die Bohrung des Gehäuses ausgebildet ist und die am proximalen Ende ausgebildete Stirnfläche des Rohrstutzens als Andruckfläche auf das Quetschteil dient und der proximale Bereich des Rohrstutzens von einem koaxial angeordneten Mantel beabstandet umgeben ist, wobei ein Ringraum gebildet ist, und auf der Innenseite des Mantels ein Gewinde oder Profilierung zum lösbaren Verbinden mit dem Gehäuse ausgebildet ist. Zum Verbinden des Schließteils mit dem Gehäuse unter Ausübung eines Quetsch- und Stauchdruckes auf das Quetschteil ist der Bund des Gehäuses so ausgebildet, daß er in den zwischen dem am Schließteil zwischen Mantel und proximalem Rohrstutzen gebildeten Ringraum einführbar ist.

Für den sicheren Sitz im Gehäuse und zur Ausübung eines Druckes auf das Quetschteil wird vorgeschlagen, daß die durchgehende Bohrung des Gehäuses zumindest vom distalen Ende desselben ausgehend stufenförmig sich verjüngend zur Mitte des Gehäuses hin ausgebildet ist und an dem stufenförmigen Absatz eine Auflagerfläche für das Quetschteil gebildet ist.

Das Quetschteil selbst kann aus einem elastischen Material wie einem thermoplastischen und/oder elastomeren Kunststoff, Silikon, Gummi oder Kautschuk gefertigt sein mit einer durchgehenden Bohrung, so daß bei Ausübung eines entsprechenden Druckes eine Stauchung erfolgen kann, wodurch der durch die Bohrung hindurchgeführte Führungsdraht durch Verkleinerung des Bohrungsdurchmessers festgeklemmt wird.

Es ist aber auch möglich, das Quetschteil aus einem harten Material wie hartem Kunststoff oder Metall zu fertigen, das nicht zusammendrückbar ist, jedoch durch entsprechende Formgebung, beispielsweise Ausbildung von Schlitzen, federnd zusammendrückbar ausgebildet ist, wodurch ebenfalls durch Verkleinerung des Durchmessers einer durchgehenden Bohrung ein Führungsdraht in denselben festklemmbar ist.

Eine vorteilhafte Ausführung der Erfindung sieht vor, daß an dem distalen Ende der Fixierungsvorrichtung ein Katheteradapter mit Katheterkonnektor lösbar befestigt ist oder an dem distalen Ende der Fixierungsvorrichtung ein Katheterkonnektor angeformt ist und an dem Katheterkonnektor ein Katheter konnektiert ist und die Position des Katheters in bezug auf die Fixierungsvorrichtung festgelegt ist und der durch die Fixierungsvorrichtung geführte Führungsdraht durch Befestigen des Schließteils an dem Gehäuse in dem Quetschteil eingequetscht und in seiner Position in bezug auf die Fixierungsvorrichtung und den an derselben befestigten Katheter festgelegt ist und eine gleichzeitige gemeinsame Positionsveränderung von Katheter und Führungsdraht unter Beibehaltung der vorher festgelegten Position des Führungsdrahtes zum Katheter durchführbar ist.

Somit ist es möglich, die erfindungsgemäße Fixierungsvorrichtung für den Führungsdraht in Verbindung mit dem Katheteradapter zum gleichzeitigen Festhalten eines Führungsdrahtes und des Katheters einzusetzen und eine gemeinsame Bewegung in axialer Richtung - vorwärts, rückwärts - durchzuführen, wobei ein Gegeneinanderverschieben von Führungsdraht und Katheter in axialer Richtung verhindert ist.

Die Anwendung der Erfindung ist beispielsweise für gastrale und jejunale Sonden mit offener Spitze oder offenem distalen Ende vorteilhaft. Gastrale und jejunale Sonden werden auch mit geschlossener Spitze hergestellt, damit der Führungsdraht beim Vorschieben nicht über das distale Ende der Sonde unerwünscht austreten kann. Eine geschlossene Sondenspitze/Katheterspitze am distalen Ende hat jedoch den Nachteil, daß Medikamentengaben an der Sondenspitze teilweise zurückgehalten werden können, da sie nur durch die seitlichen Augen der Sonde austreten können, wodurch die Medikamententherapie beispielsweise verfälscht werden kann.

Die Erfindung wird nachfolgend anhand der Zeichnung an einem Ausführungsbeispiel näher erläutert. Es zeigen:
- Figur 1a: eine Fixierungsvorrichtung für einen Führungsdraht und einen Katheter im Längsschnitt in geschlossener Position mit separatem Katheteradapter mit Katheterkonnektor,
- Figur 1b: einen Fixierungsvorrichtung für einen Führungsdraht und einen Katheter im Längsschnitt in geschlossener Position mit integriertem Katheteradapter,
- Figur 2: eine Vorrichtung zum Halten und Führen eines Führungsdrahtes gemäß Stand der Technik in schematischer Form,
- Figur 3: die Fixierungsvorrichtung für den Führungsdraht und den Katheter gemäß der Erfindung in Explosionsdarstellung im Längsschnitt gemäß Figur 1 a in geöffneter Position mit Katheteradapter,
- Figur 4: die Fixierungsvorrichtung für das Halten des Führungsdrahtes gemäß Figur 3 in geschlossener Position ohne Katheteradapter,
- Figuren 5 und 6: zwei verschiedene Ausführungsbeispiele des Quetschteils in drei Ansichten,
- Figur 7: das Gehäuse gemäß Figur 4 in verkleinerter Darstellung in Draufsicht,
- Figuren 8 und 9: zwei weitere Ausführungsbeispiele eines Katheteradapters zum Verbinden mit der Fixierungsvorrichtung gemäß Figur 4.

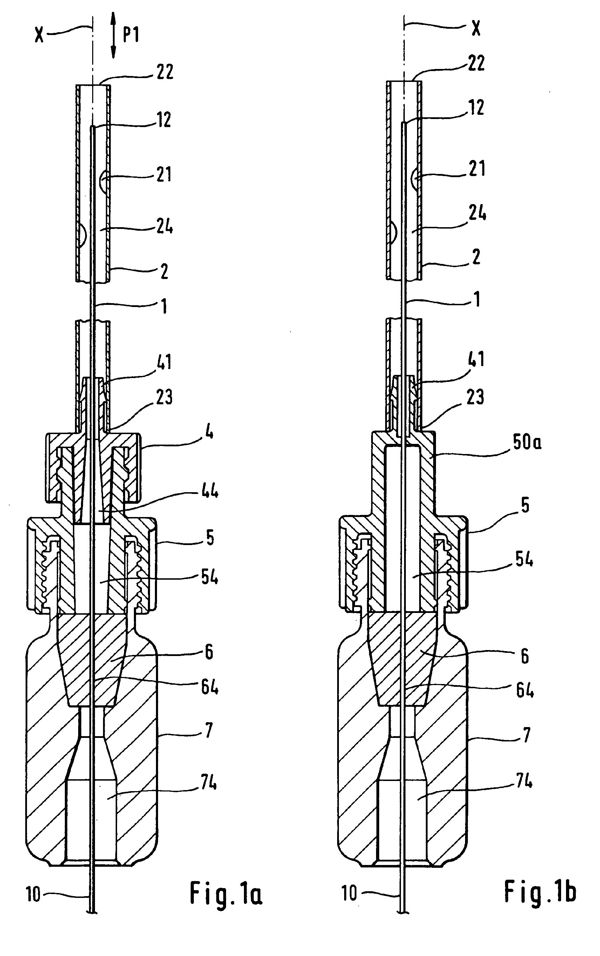

Die erfindungsgemäße Vorrichtung zum Fixieren und Führen eines Führungsdrahtes 1 und eines Katheters 2 ist in der Figur 1a im zusammengebauten Zustand und mit fest eingeklemmtem, d. h. in seiner Position festgelegtem Führungsdraht 1 dargestellt. Das proximale vom Patienten abgewandte Ende des Führungsdrahtes ist mit 10 bezeichnet, das distale Ende des Führungsdrahtes mit 12. Das Gehäuse 17, das Quetschteil 6 und das Schließteil 5 bilden die Fixierungsvorrichtung zum Halten - Festklemmen - des Führungsdrahtes und zum Führen des Führungsdrahtes. Die Fixierungsvorrichtung umfaßt das Gehäuse 7 mit der in axialer Richtung X durchgehenden Bohrung 74 und dem in die Bohrung des Gehäuses 7 bereichsweise eingesetzten Quetschteil 6, das ebenfalls eine axial durchgehende Bohrung 64 aufweist. Das Quetschteil 6 wird in dem Gehäuse 7 mittels des Schließteils 5 fixiert, das unter Ausübung eines axialen Druckes auf das Quetschteil 6 lösbar an dem Gehäuse 7 befestigt ist. Auch das Schließteil 5 weist eine durchgehende axiale Bohrung 54 auf, die den Führungsdraht 1 aufnimmt. An dem distalen Ende des Schließteils 5 schließt ein Katheteradapter 4 an mit angeformtem Katheterkonnektor 41, der ebenfalls eine axial durchgehende Bohrung 44 aufweist für die Durchleitung des Führungsdrahtes 1. Der Katheteradapter 4 ist an dem Schließteil 5 lösbar befestigt. An dem Katheterkonnektor 41 des Katheteradapters 4, der als Schlauchtülle 41 ausgebildet ist, ist der Katheter 2 konnektiert. Der Katheter 2 weist ein von seinem proximalen Ende 23 bis zum distalen Ende 22 durchgehendes Lumen 24 auf, der Katheter kann aber auch eine geschlossene distale Spitze aufweisen. Der Katheter 2 ist mit seinem proximalen Ende 23 am Katheterkonnektor 41 des Katheteradapters 4 befestigt. Der Führungsdraht 1 ist ebenfalls in den Katheter bis zu dessen distalem Endbereich eingeführt. Die erfindungsgemäße Fixierungsvorrichtung für den Führungsdraht 1 und den Katheter 2 gemäß Figur 1a dient der Konnexion und Befestigung des Katheters 2 und der gleichzeitigen Fixierung und Befestigung des Führungsdrahtes 1 an der aus Gehäuse 7, Quetschteil 6, Schließteil 5 und Katheteradapter 4 zusammengesetzten Fixierungsvorrichtung. Das Gehäuse dient als Griff und ermöglicht eine einheitliche Handhabung. Die Fixierungsvorrichtung mit Katheteradapter ermöglicht die gleichzeitige Positionsveränderung von Katheter und Führungsdraht, wobei die Position des Führungsdrahtes vorher in dem Katheter festlegbar ist. Hierbei ist es möglich, insbesondere das distale Ende des Katheters zum distalen Ende des Führungsdrahtes vorher festzulegen durch eine starre Kopplung von Führungsdraht 1 und Katheter 2 mittels der Fixierungsvorrichtung. Bei einer nachher erfolgenden gleichzeitigen und gemeinsamen Bewegung von Katheter und Führungsdraht in Pfeilrichtung P1 mittels der Vorrichtung wird ein axiales Verschieben des Führungsdrahtes in bezug auf den Katheter vermieden.

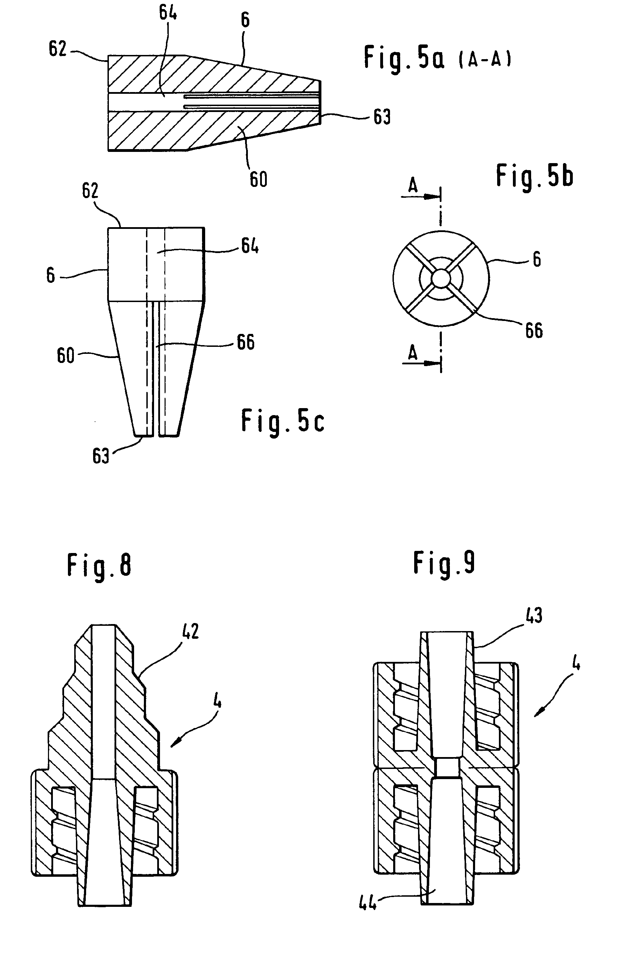

Anstelle des in der Figur 1 a dargestellten Katheteradapters 4 mit angeformter Schlauchtülle ist es auch möglich, beispielsweise einen wie in der Figur 8 dargestellten Katheteradapter mit Katheterkonnektor, der als Stufenadapter mit Stufenkonnektor 42 ausgebildet ist, oder einen Katheteradapter mit Katheterkonnektor gemäß Figur 9, der als Doppeladapter mit Luer-Lock-Konnektor ausgebildet ist, einzusetzen.

Es ist auch möglich, das Schließteil 5 an seinem distalen Endbereich integral mit einem Katheterkonnektor auszubilden, wie in der Figur 1b dargestellt. Es ist kein separater Katheteradapter 4 wie bei Figur 1a vorgesehen, sondern das distale Ende 50a des Schließteils 5 ist direkt als Konnektor für den Katheter 2 ausgebildet, beispielsweise als Schlauchtülle 41 oder als Stufenkonnektor oder als männlicher oder weiblicher mit Luer-Lock-Konnektor.

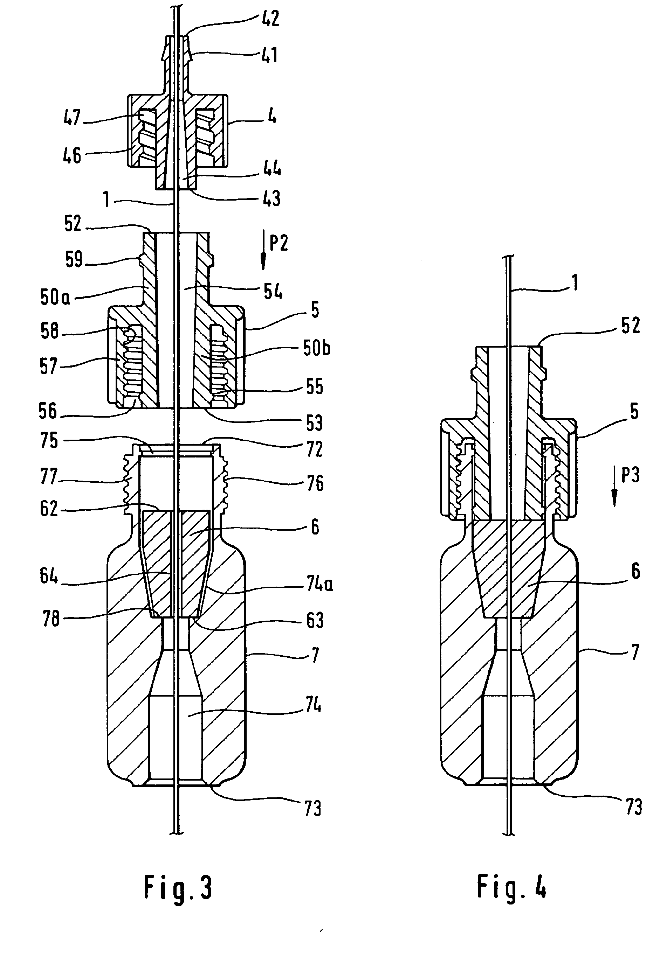

In der Figur 3 sind die zu der Fixierungsvorrichtung für die Halterung und Führung sowohl des Führungsdrahtes als auch des Katheters erfindungsgemäß vorgesehenen Teile in Explosionsdarstellung dargestellt und werden anhand dieser nachfolgend näher erläutert. Hierbei ist der Führungsdraht noch nicht fixiert - geöffnete Position.

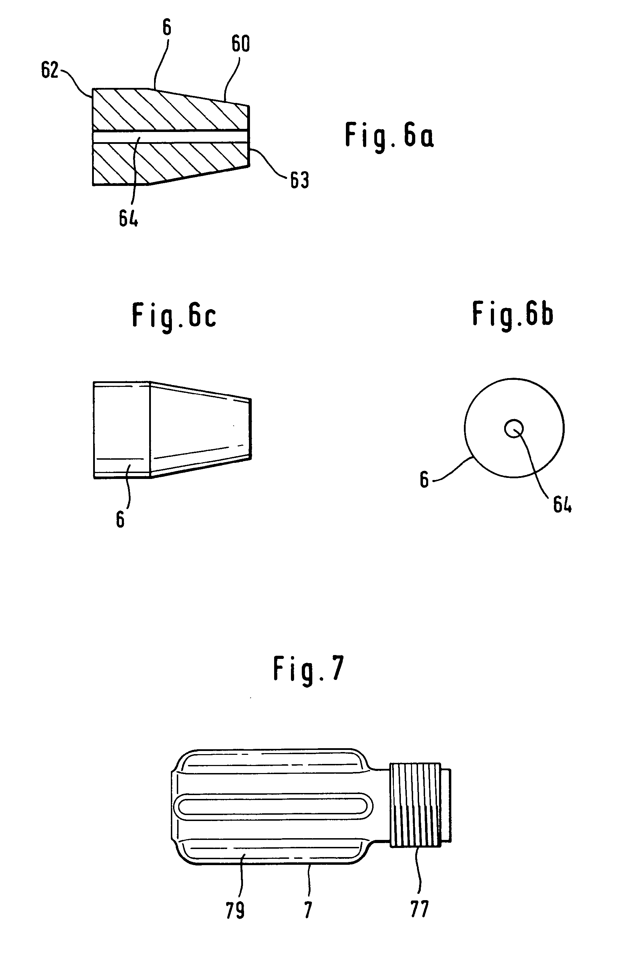

Das Gehäuse 7 weist eine von seinem proximalen Ende 73 bis zum distalen Ende 72 durchgehende Bohrung 74 auf, die vom distalen Ende 72 ausgehend bis etwa zur Mitte hin sich stufenweise verjüngt und hier an dem gebildeten Absatz eine Auflagerfläche 78 für das Quetschteil 6 bildet. Der im distalen Endbereich 72 des Gehäuses 7 ausgebildete Bund 77 weist einen kleineren Außendurchmesser als der sonstige Körper des Gehäuses 7 auf und ist außenseitig beispielsweise mit einem Gewinde 76 versehen zum Verschrauben mit dem Schließteil 5. Das Gehäuse 7 dient zugleich als Griff für die Fixierungsvorrichtung und kann, wie beispielsweise in der Figur 7 dargestellt, außenseitig mit mehreren sich in axialer Richtung erstreckenden und gleichmäßig voneinander beabstandeten Rippen 79 ausgebildet sein, welche ein sicheres Anfassen ermöglichen und ein Abrutschen vermeiden. Das Gehäuse 7, das als Griff dient, kann jedoch auch noch andere Formen aufweisen. Für eine gute Handhabung sollte der Durchmesser des Gehäuses 7 / Griff wenigstens etwa 12 mm betragen und der verdickte Teil des Gehäuses 7 eine Länge von mindestens 15 mm aufweisen. Das Gehäuse 7, das Schließteil 5 und auch der Katheteradapter 4 werden bevorzugt aus einem thermoplastischen Kunststoff als Spritzgußteil hergestellt.

Die Bohrung 74 des Gehäuses 7 verjüngt sich zu der Auflagerfläche 78 über den Bereich 74a. In diesem Bereich wird das mit einer entsprechenden äußeren Kontur ausgestattete in dem Beispiel elastische Quetschteil 6 eingesetzt. Das Quetschteil 6 kann beispielsweise aus einem elastischen und/oder thermoplastischen Kunststoff gefertigt sein und ist beispielhaft in der Figur 6 in einem Längsschnitt, Draufsicht und Seitenansicht dargestellt. Das Quetschteil 6 liegt mit seinem proximalen Ende 63 an der Auflagerfläche 78 des Gehäuses 7 an und endet mit seinem distalen Ende 62 noch innerhalb der Bohrung 74 des Gehäuses 7. Das Quetschteil 6 weist eine zentrale axial durchgehende Bohrung 64 vom distalen Ende zum proximalen Ende für die Durchführung des Führungsdrahtes 1 auf, wie in Figur 3 ersichtlich. Das Schließteil 5 ist als Anschlußteil und Stempel für das Quetschteil ausgebildet. Wenn das Schließteil 5 in Pfeilrichtung P2, P3 auf den Bund 77 des Gehäuses 7 aufgeschraubt wird, wird hierbei das Quetschteil 6, wie aus Figur 4 ersichtlich, axial gestaucht, wodurch die Bohrung 64 des Quetschteils 6 sich verschließt und der in der Bohrung 64 sich befindende Führungsdraht 1 wird in dem Quetschteil 6 festgeklemmt. Das Schließteil 5 weist einen durchgehenden Rohrstutzen mit einer vom distalen Ende 52 bis zum proximalen Ende 53 durchgehenden Bohrung 54 auf. Das distale Ende des Rohrstutzens 50a des Schließteils 5 ist als Luer-Lock-Anschluß ausgebildet und weist außenseitig vorstehende Wülste 59 zum Verrasten mit dem Katheteradapter 4 auf. Zum proximalen Ende hin ist der Rohrstutzen 50b ebenfalls außenseitig mit einer Rastwulst 55 ausgestattet, des weiteren wird er von dem koaxial ausgebildeten Mantel 57 unter Ausbildung des Ringraumes 56 umgeben. An der Innenseite des Mantels 57 ist ein Innengewinde 58 ausgebildet, so daß das Schließteil 5 mit dem Ringraum 56 über den Bund 77 des Gehäuses 7 geführt und auf diesen aufgeschraubt werden kann, wie auch aus Figur 4 ersichtlich. Nahe dem distalen Ende 72 des Gehäuses 7 ist am Bund 77 innenseitig eine Ringwulst 75 ausgebildet, über die das Schließteil 5 mit der Rastwulst 55 rastet, so daß zwar die durch Verschraubung erzeugbare starre Verbindung zwischen Schließteil 5 und Gehäuse 7 lösbar ist, aber Gehäuse und Schließteil über Ringwulst 75 und Rastwulst 55 beweglich aneinanderhängen.

Für die Konnexion des Katheters 2 wird an der distalen Seite 52 des Schließteils 5 der Katheteradapter 4 mit Katheterkonnektor 41 lösbar befestigt. Der Katheteradapter 4 weist wiederum ein durchgehendes Lumen 44 vom proximalen Ende 43 zum distalen Ende 42 auf. Der Anschluß ist zur proximalen Seite 43 als Luer-Lock-Anschluß 46, 47 ausgebildet und zur distalen Seite hin als Schlauchtülle 41. Die Montagerichtung ist mit P2 in der Figur 3 bezeichnet und es werden alle Teile 4, 5, 6, 7 auf den Führungsdraht 1 aufgefädelt und dann wird zuerst das Quetschteil 6 in das Gehäuse 7 eingesetzt, daraufhin das Schließteil 5 aufgeschraubt und das Quetschteil 6 zusammengequetscht und der Führungsdraht 1 fixiert, danach wird der Katheteradapter 4 an dem Schließteil 5 befestigt, wenn dies nicht bereits vorher geschehen war. Nunmehr kann der Katheter 2, wie in der Figur 1 dargestellt, mit seinem proximalen Ende 23 an dem Katheterkonnektor 41 des Katheteradapters 4 befestigt werden.

In der Figur 5 ist noch eine weitere Möglichkeit der Ausbildung eines Quetschteils 6 dargestellt, das aus einem harten Werkstoff gefertigt ist und mit zusätzlichen Schlitzen 67 im Bereich des konischen Teils 60 versehen ist, wodurch eine Zusammendrückbarkeit und Anpreßkraft für den Führungsdraht in dem Quetschteil 6 erzielbar ist.

## Patentansprüche

1. Vorrichtung zum Halten und Führen eines Führungsdrahtes in einem Katheter, welche an dem Führungsdraht lösbar befestigbar ist, mit einem proximalen und einem distalen Ende und mit einer vom proximalen Ende bis zum distalen Ende der Vorrichtung durchgehenden Ausnehmung für die Durchführung des Führungsdrahtes zum Einführen des Katheters in Gefäße oder Körperhöhlen eines Patienten, **dadurch gekennzeichnet, daß** sie Mittel zum lösbaren Befestigen des Katheters aufweist, so daß eine gemeinsame Bewegung des Führungsdrahtes und des Katheters bei vorher festgelegter Position des Führungsdrahtes in bezug auf den Katheter mittels der Vorrichtung ausführbar ist.

2. Vorrichtung nach Anspruch 1, **dadurch gekennzeichnet, daß** sie eine Fixierungsvorrichtung zum Durchführen und Fixieren des Führungsdrahtes, umfassend ein Gehäuse, ein in das Gehäuse einsetzbares Quetschteil und ein mit dem Gehäuse lösbar verbindbares Schließteil, aufweist und die Mittel zum lösbaren Befestigen des Katheters am distalen Ende der Fixierungsvorrichtung angeordnet sind.

3. Vorrichtung nach Anspruch 1 oder 2, **dadurch gekennzeichnet, daß** sie als Mittel zum Befestigen des Katheters mit einem am distalen Ende der Fixierungsvorrichtung lösbar befestigbaren Katheteradapter mit Katheterkonnektor für die Konnexion des Katheters ausgerüstet ist.

4. Vorrichtung nach Anspruch 1 oder 2, **dadurch gekennzeichnet, daß** als Mittel zur Befestigung des Katheters die Fixierungsvorrichtung an ihrem distalen Ende integral mit einem Katheterkonnektor für die Konnexion des Katheters ausgebildet ist.

5. Vorrichtung nach einem der Ansprüche 1 bis 4, **dadurch gekennzeichnet, daß** der Katheterkonnektor als Schlauchtülle ausgebildet ist.

6. Vorrichtung nach einem der Ansprüche 1 bis 4, **dadurch gekennzeichnet, daß** der Katheterkonnektor als Luer-Lock-Konnektor ausgebildet ist.

7. Vorrichtung nach einem der Ansprüche 1 bis 4, **dadurch gekennzeichnet, daß** der Katheterkonnektor als Stufenkonnektor ausgebildet ist.

8. Vorrichtung nach einem der Ansprüche 1 bis 7, **dadurch gekennzeichnet, daß** die Fixierungsvorrichtung für den Führungsdraht ein als Griff ausgebildetes Gehäuse mit einer durchgehenden Bohrung zum Durchführen des Führungsdrahtes, ein Quetschteil mit einer durchgehenden Bohrung zum Durchführen des Führungsdrahtes und ein als Stempel zum Einwirken auf das Quetschteil ausgebildetes Schließteil mit einer durchgehenden Bohrung umfaßt, wobei das Quetschteil in die Bohrung des Gehäuses einsetzbar ist und das Schließteil an dem Gehäuse unter Ausübung eines Druckes auf das Quetschteil lösbar befestigbar ist, wobei die Bohrungen des Gehäuses und des Quetschteiles und des Schließteils sich in einer gemeinsamen Achse erstrecken.

9. Vorrichtung nach Anspruch 8, **dadurch gekennzeichnet, daß** das distale Ende des Schließteils mit einem Anschluß, wie Luer-Lock-Anschluß für die Konnexion des Katheteradapters oder des Katheters ausgebildet ist.

10. Vorrichtung nach einem der Ansprüche 7 bis 9, **dadurch gekennzeichnet, daß** das Schließteil an seinem proximalen Ende mit dem Gehäuse mittels einer Schraubverbindung, mittels eines Bajonettverschlusses und/oder mittels einer Rast- und/oder Klemmverbindung lösbar verbindbar ist.

11. Vorrichtung nach einem der Ansprüche 7 bis 10, **dadurch gekennzeichnet, daß** das Schließteil einen von seinem proximalen Ende bis zum distalen Ende durchgehenden Rohrstutzen mit durchgehender Bohrung aufweist und der proximale Endbereich des Rohrstutzens zum Einführen in die Bohrung des Gehäuses ausgebildet ist und die am proximalen Ende ausgebildete Stirnfläche des Rohrstutzens als Andruckfläche auf das Quetschteil dient und der proximale Bereich des Rohrstutzens von einem koaxial angeordneten Mantel beabstandet umgeben ist, der auf seiner Innenseite mit einem Gewinde oder Profilierung zum lösbaren Verbinden mit dem Gehäuse ausgebildet ist.

12. Vorrichtung nach einem der Ansprüche 7 bis 11, **dadurch gekennzeichnet, daß** das Gehäuse zylindrisch oder ballig ausgebildet ist und außenseitig zur Verbesserung der Griffigkeit mit einer Profilierung wie axial verlaufenden vorstehenden gleichmäßig über den Umfang verteilt angeordneten Rippen oder eine Riffelung ausgebildet ist.

13. Vorrichtung nach einem der Ansprüche 7 bis 12, **dadurch gekennzeichnet, daß** der distale Endbereich des Gehäuses als abgesetzter mit gegenüber dem übrigen Gehäuse kleinerem Durchmesser versehener Bund ausgebildet ist, wobei der Bund außenseitig eine Profilierung wie Gewinde, Nuten, Wülste und/oder Rillen zur Ausbildung eines Formschlusses mit dem Schließteil aufweist.

14. Vorrichtung nach einem der Ansprüche 7 bis 13, **dadurch gekennzeichnet, daß** der Bund in den zwischen dem am Schließteil zwischen Mantel und proximalem Rohrstutzen gebildeten Ringraum einführbar ist.

15. Vorrichtung nach einem der Ansprüche 7 bis 14, **dadurch gekennzeichnet, daß** die durchgehende Bohrung des Gehäuses zumindest vom distalen Ende desselben ausgehend stufenförmig sich verjüngend zur Mitte des Gehäuses hin ausgebildet ist und an dem stufenförmigen Absatz eine Auflagerfläche für das Quetschteil gebildet ist.

16. Vorrichtung nach einem der Ansprüche 7 bis 15, **dadurch gekennzeichnet, daß** das Quetschteil aus elastischem Material wie einem thermoplastischen und/oder elastomeren Kunststoff, Silikon, Gummi oder einem Kautschuk gefertigt ist.

17. Vorrichtung nach einem der Ansprüche 7 bis 15, **dadurch gekennzeichnet, daß** das Quetschteil aus einem harten Material wie hartem Kunststoff oder Metall gefertigt ist und federnd ausgebildet und zusammendrückbar ist.

18. Vorrichtung nach einem der vorangehenden Ansprüche 1 bis 17, **dadurch gekennzeichnet, daß** an dem distalen Ende der Fixierungsvorrichtung ein Katheteradapter mit Katheterkonnektor lösbar befestigt ist oder an dem distalen Ende der Fixierungsvorrichtung ein Katheterkonnektor angeformt ist und an dem Katheterkonnektor ein Katheter konnektiert ist und die Position des Katheters in bezug auf die Fixierungsvorrichtung festgelegt ist und der durch die Fixierungsvorrichtung geführte Führungsdraht durch Befestigen des Schließteils an dem Gehäuse in dem Quetschteil eingequetscht und in seiner Position in bezug auf die Fixierungsvorrichtung und den an derselben befestigten Katheter festgelegt ist und eine gleichzeitige gemeinsame Positionsveränderung von Katheter und Führungsdraht unter Beibehaltung der vorher festgelegten Position des Führungsdrahtes zum Katheter durchführbar ist.
